(19) Europäisches Patentamt
European Patent Office
Office européen des brevets

(11) **EP 3 365 462 B1**

(12) **EUROPEAN PATENT SPECIFICATION**

(45) Date of publication and mention
of the grant of the patent:
**29.09.2021 Bulletin 2021/39**

(51) Int Cl.:
***C12Q 1/6883*** (2018.01)   ***C12Q 1/689*** (2018.01)

(21) Application number: **16787529.3**

(22) Date of filing: **20.10.2016**

(86) International application number:
**PCT/GB2016/053269**

(87) International publication number:
**WO 2017/068347 (27.04.2017 Gazette 2017/17)**

(54) **DETECTION OF BACTERIAL INFECTION**

NACHWEIS BAKTERIELLER INFEKTIONEN

DÉTECTION D'UNE INFECTION BACTÉRIENNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.10.2015 GB 201518597**

(43) Date of publication of application:
**29.08.2018 Bulletin 2018/35**

(73) Proprietor: **The University of Liverpool Liverpool, Merseyside L69 7ZX (GB)**

(72) Inventor: **GRIFFITHS, Michael Liverpool Merseyside L69 7ZX (GB)**

(74) Representative: **HGF 1 City Walk Leeds LS11 9DX (GB)**

(56) References cited:
**WO-A1-2012/038541     WO-A1-2015/155517
US-A1- 2009 221 021     US-A1- 2014 323 391**

- **MARGIT LILL ET AL: "Peripheral blood RNA gene expression profiling in patients with bacterial meningitis", FRONTIERS IN NEUROSCIENCE, vol. 7, 18 March 2013 (2013-03-18), XP055330725, CH ISSN: 1662-4548, DOI: 10.3389/fnins.2013.00033**
- **RAQUEL ALMANSA ET AL: "Critical COPD respiratory illness is linked to increased transcriptomic activity of neutrophil proteases genes", BMC RESEARCH NOTES, BIOMED CENTRAL LTD, GB, vol. 5, no. 1, 2 August 2012 (2012-08-02), page 401, XP021108156, ISSN: 1756-0500, DOI: 10.1186/1756-0500-5-401**
- **CHLOE I. BLOOM ET AL: "Transcriptional Blood Signatures Distinguish Pulmonary Tuberculosis, Pulmonary Sarcoidosis, Pneumonias and Lung Cancers", PLOS ONE, vol. 8, no. 8, 5 August 2013 (2013-08-05), page e70630, XP055330577, DOI: 10.1371/journal.pone.0070630**
- **MATTHEW P. R. BERRY ET AL: "An interferon-inducible neutrophil-driven blood transcriptional signature in human tuberculosis", NATURE, vol. 466, no. 7309, 19 August 2010 (2010-08-19), pages 973-977, XP055001768, ISSN: 0028-0836, DOI: 10.1038/nature09247**
- **KORKMAZ ET AL: "Neutrophil elastase, proteinase 3 and cathepsin G: Physicochemical properties, activity and physiopathological functions", BIOCHIMIE, MASSON, PARIS, FR, vol. 90, no. 2, 25 October 2007 (2007-10-25), pages 227-242, XP022438298, ISSN: 0300-9084, DOI: 10.1016/J.BIOCHI.2007.10.009**
- **KORKMAZ BRICE ET AL: "Neutrophil elastase, proteinase 3, and cathepsin G as therapeutic targets in human diseases", PHARMACOLOGICAL REVIEWS, AMERICAN SOCIETY FOR PHARMACOLOGY AND EXPERIMENTAL THERAPEUTICS, US, vol. 62, no. 4, 1 December 2010 (2010-12-01), pages 726-759, XP009152862, ISSN: 0031-6997**

Remarks:
    The file contains technical information submitted after the application was filed and not included in this specification

Remarks:
    The file contains technical information submitted after the application was filed and not included in this specification

**Description**

**FIELD OF THE INVENTION**

The present invention relates to methods of detecting a bacterial infection of the blood stream or central nervous system, such as bacterial meningitis. The invention provides the medical use of antibacterial agents in the treatment of a bacterial infection of the blood stream or central nervous system, such as bacterial meningitis, in a subject identified as having such a bacterial infection through use of a method of the invention.

**INTRODUCTION**

Bacterial infection remains a significant cause of morbidity and mortality in UK and worldwide. Bacterial infection can involve body compartments, such as the brain, lungs, gut. The infection can also involve the whole body.

Bacterial infection of the brain, known clinically as meningo-encephalitis or meningitis (because of associated inflammation of the brain and/or membranes [meninges] that cover the brain), is a catastrophic infection linked with high rates of death or disability Bacterial meningitis requires emergency treatment with antibiotics and other adjunctive therapies. It is a significant cause of morbidity and mortality in the UK and worldwide . The health economic costs of bacterial meningitis are disproportionately large because many patients are left with neurological disability; the costs to society of a single case can be as high as £3-5M (Meningitis Research Foundation data).

Currently, meningitis is difficult to diagnose because, it requires a lumbar puncture (LP), to obtain a sample of cerebrospinal fluid (CSF) surrounding the brain. In many patients, the LP is not performed when needed: for some, because the patient is too critically ill, with acute seizures or coma; for others LP is delayed whilst brain scans are performed, or because of a lack of trained staff. Consequently, clinicians can start antibiotics which may later prove unnecessary, or delay starting antibiotics until all investigations are done. Delays in diagnosis or in starting anti-microbial therapy can lead to a worse patient outcome including increased mortality.

Current methods for detecting bacterial meningitis include blood cultures and lumbar puncture. Blood cultures often remain negative for a long period of time if the disease is caused by slow growing bacteria, thereby delaying an accurate diagnosis and the commencement of an optimised antibiotic. Performing a lumbar puncture on the other hand, is not suitable in all subjects, for example those who have recently taken blood thinning medication. It is also associated with an unnecessary risk and discomfort.

Additionally, subjects suspected of having bacterial meningitis are typically given empirical antibiotic treatment, despite the lack of results confirming the presence of bacterial meningitis. This often leads to unnecessary treatment with antibiotics, which increases the cost of treatment and may lead to the development of antibiotic resistance.

A previous study by Lill *et al* (2003) utilises a method of detecting bacterial meningitis by assaying a blood sample for the expression of a pair of at least two genes to obtain data indicative of the relative abundance of particular target molecules. Additionally, in patent application WO 2015/155517, a method of detecting a bacterial infection (sepsis) is disclosed, involving assaying a blood sample for expression levels of particular genes and determining the presence of a bacterial infection (sepsis). Another method of detecting a bacterial infection (e.g. a bacterial infection of the blood stream and/or *S.aureus* infection) by analysing a blood sample for the expression of at least two genes was disclosed in patent application US 2014/0323391. The inventors of the present invention have shown that the ratio of the abundance of expression level of particular gene pairs provides sufficient sensitivity and specificity for diagnosing bacterial infections of the blood stream and central nervous system.

**SUMMARY OF THE INVENTION**

In a first aspect, the invention provides a method of detecting a bacterial infection of the blood stream or central nervous system in a subject, the method comprising:

- assaying a blood sample representative of gene expression in the subject to obtain data indicative of the relative abundance ratio of target molecules indicative of the expression of the gene pair: ELANE and IFI44L, and optionally at least one further gene pair from the group consisting of: CTSG and IFI44L; ELANE and S1PR5; CTSG and S1PR5; GRK6 and TXLNA; DUSP1 and IFI44L; APMAP and KMT2A; DUSP1 and HP1BP3; FLOT1 and NMT1; APMAP and HUWE1; ELANE and SIGLEC1; ELANE and IF127; and DUSP1 and NMT1;
- wherein the target molecules are selected from: an mRNA target molecule comprising an RNA transcript of the relevant gene, and a protein target molecule comprising a protein encoded by the relevant gene; and
- determining the presence of a bacterial infection of the blood stream or central nervous system in the subject in dependence on the data.

Suitably, a method in accordance with the first aspect of the invention may comprise obtaining data indicative of the relative abundance ratio of target molecules in respect of each of the gene pairs in the group consisting of: ELANE and IFI44L; CTSG and IFI44L; ELANE and S1PR5; and CTSG and S1PR5; GRK6 and TXLNA; DUSP1 and IFI44L; APMAP and KMT2A ; DUSP1 and HP1BP3; APMAP and HUWE1; or DUSP1 and NMT1.

Suitably, a method in accordance with the first aspect of the invention, in which a bacterial infection is detected in the subject, further comprises conducting a further confirmatory test for the bacterial infection in the subject.

In an embodiment the invention provides a method in accordance with the first aspect of the invention, wherein the bacterial infection of the central nervous system is caused by bacteria selected from the group consisting of: *Streptococcus pneumoniae, Neisseria meningitidis, Haemophilus influenzae; Streptococcus agalactiae; Listeria monocytogenes; Escherichia coli; Mycobacterium tuberculosis; and Staphylococcus aureus.*

In an embodiment the invention provides a method in accordance with the first aspect of the invention, wherein the target molecule is an mRNA target molecule, and wherein the assay is selected from the group consisting of: RT-PCR, real-time PCR, Northern blot, RNA sequencing (RNA-seq) and RNA microarray.

In an embodiment the invention provides a method in accordance with the first aspect of the invention, wherein the target molecule is a protein target molecule, and wherein the assay is selected from the group consisting of: ELISA, radioimmunoassay, immunoprecipitation, Western blot and mass spectrometry.

In an embodiment of the first aspect of the invention, there is provided a method, wherein the subject has symptoms consistent with a bacterial infection such as bacterial meningitis, or wherein the subject lacks some or all symptoms consistent with a bacterial infection such as bacterial meningitis.

In an embodiment of the first aspect of the invention wherein the subject is believed to be at risk of developing bacterial meningitis.

In an embodiment of the first aspect of the invention, there is provided a method wherein the step of determining comprises using the data to calculate one or more functions, where the presence of a bacterial infection in the subject is determined to be present if one or more of the calculated functions satisfies a predetermined condition.

In an embodiment of the first aspect of the invention, there is provided a method wherein the one or more functions includes a logical function or a linear function.

In an embodiment of the first aspect of the invention, there is provided a method, wherein the linear function is a linear discriminant function, and optionally wherein the predetermined condition is satisfied if the calculated linear discriminant function exceeds a predetermined threshold.

In a second aspect the invention provides an antibacterial agent for use in the treatment of a bacterial infection of the central nervous system in a subject identified as having a bacterial infection of the central nervous system by a method in accordance with the first aspect of the invention.

In an embodiment of the second aspect of the invention, the bacterial infection is bacterial meningitis.

## SUBJECT MATTER DISCLOSED HEREIN

The subject matter disclosed herein does not form part of the invention. The terms "disclosure" and "example" as disclosed herein refer to information that may be useful in the context of the present invention but do not form part of the claimed invention.

In the present disclosure, there is provided a method of detecting a bacterial infection in a subject, the method comprising:

- assaying a sample representative of gene expression in the subject to obtain data indicative of the relative abundance of target molecules indicative of the expression of at least one gene pair from the group consisting of: ELANE and IF144L; CTSG and IFI44L; ELANE and S1PR5; and CTSG and S1PR5; and
- determining the presence of a bacterial infection in the subject in dependence on the data.

In another disclosure, a method may comprise obtaining data indicative of the relative abundance of target molecules in respect of two or more gene pairs from those listed. For example, a method of the first aspect of the invention may comprise obtaining data indicative of the relative abundance of target molecules in respect of three or the gene pairs listed, or of all four gene pairs listed. Indeed, embodiments of the first aspect of the invention that obtain data indicative of the relative abundance of target molecules in respect of the four gene pairs: ELANE and IF144L; CTSG and IFI44L; ELANE and S1PR5; and CTSG and S1PR5; and determine the presence of a bacterial infection in the subject in dependence on such data represent particularly useful methods of the invention.

Another example relates to a method of detecting a bacterial infection in a subject, the method comprising:

- assaying a sample representative of gene expression in the subject to obtain data indicative of the relative abundance of target molecules indicative of the expression of at least at least one gene pair from the group consisting of: ELANE and IFI44L; CTSG and IFI44L; ELANE and S1PR5; CTSG and S1PR5; GRK6 and TXLNA; DUSP1 and IFI44L; APMAP and KMT2A; DUSP1 and HP1BP3; FLOT1 and NMT1; APMAP and HUWE1; ELANE and SIGLEC1; ELANE and IF127; and DUSP1 and NMT1; and
• determining the presence of a bacterial infection in the subject in dependence on the data.

Also disclosed herein is an example of a method of detecting a bacterial infection in accordance with the invention, in which a bacterial infection is detected in a subject, which may further comprise a step of conducting a further confirmatory test for the bacterial infection in the subject. Suitably, in such an example, a bacterial infection is diagnosed when the results of both the first test (a method of the invention) and the second test (the confirmatory test) indicate the presence of bacterial infection. For example, a sample of biological fluid, such as CSF from lumbar puncture or blood from venepuncture, may be cultured in order to grow the microorganism associated with infection. This allows confirmation of bacterial infection and also allows determination of antimicrobial susceptibility of any isolated pathogen.

The disclosure also provides an example of a method in which a bacterial infection is detected in a subject, which may further comprise a step of providing the subject with treatment for the bacterial infection.

Indeed, in another example, there is disclosed herein a method of treating a bacterial infection in a subject, the method comprising:

• assaying a sample representative of gene expression in the subject to obtain data indicative of the relative abundance of target molecules indicative of the expression of at least at least one gene pair from the group consisting of: ELANE and IF144L; CTSG and IFI44L; ELANE and S1PR5; and CTSG and S1PR5;
• determining the presence of a bacterial infection in the subject in dependence on the data; and

providing treatment for a bacterial infection to the subject when the determination indicates the presence of a bacterial infection in the subject.

Also disclosed herein is a method of treating a bacterial infection in a subject, the method comprising:

• assaying a sample representative of gene expression in the subject to obtain data indicative of the relative abundance of target molecules indicative of the expression of at least at least one gene pair from the group consisting of: ELANE and IFI44L; CTSG and IFI44L; ELANE and S1PR5; CTSG and S1PR5; GRK6 and TXLNA; DUSP1 and IFI44L; APMAP and KMT2A; DUSP1 and HP1BP3; FLOT1 and NMT1; APMAP and HUWE1; ELANE and SIGLEC1; ELANE and IF127; and DUSP1 and NMT1;
• determining the presence of a bacterial infection in the subject in dependence on the data; and

providing treatment for a bacterial infection to the subject when the determination indicates the presence of a bacterial infection in the subject.

The treatment for a bacterial infection, such as bacterial meningitis, provided in a method disclosed herein may comprise providing the subject requiring such treatment with a therapeutically effective amount of an antibacterial agent.

Methods disclosed herein may comprise obtaining data indicative of the relative abundance of target molecules indicative of the expression further gene pairs, as long as this is in addition to obtaining data indicative of relative abundance of at least one of the gene pairs referred to in this section above.

Suitable examples of further gene pairs, and the benefits that may be gained by employing such further pairs, are described elsewhere in the present disclosure.

Merely by way of example, further gene pairs particularly suitable for use in the methods disclosed herein may include at least one of: GRK6 and TXLNA; DUSP1 and IFI44L; C20ORF3 and MLL; DUSP1 and HP1BP3; ELANE and SIGLEC1; or ELANE and IFI27.

Also disclosed herein is a method of detecting the relative abundance of target molecules in a subject suspected of having or developing a bacterial infection, the method comprising:

• Providing a sample representative of gene expression in the subject suspected of having or developing a bacterial infection;
• Detecting the relative abundance of target molecules indicative of the expression of at least one gene pair, wherein the gene pair is selected from the group according to any of the first, second, third or fourth aspects of the invention.

Examples disclosed in respect of one method should also be taken as applicable to the other methods disclosed herein, unless incompatible, or otherwise stated.

## DETAILED DESCRIPTION

The methods of the present disclosure provide simple tests that may be used in the diagnosis of bacterial infections. Such infections include, but are not limited to, bacterial meningitis. The advantages provided in respect of bacterial meningitis are notable, and worthy of further comment at this time.

As discussed elsewhere in the specification, the methods of the invention may be put into practice in the form of a blood test. The use of a blood sample to detect bacterial infections of the brain, such as bacterial meningitis, provides advantages in that it avoids the need for the more invasive lumbar punctures generally used in accepted prior art techniques for the diagnosis of bacterial meningitis.

As described herein, a sample of biological fluid, such as CSF from lumbar puncture or blood from venepuncture, may be cultured in order to grow the microorganism associated with infection. This allows confirmation of bacterial infection and also allows determination of antimicrobial susceptibility of any isolated pathogen. The disadvantage of culture is that it typically takes 48 hours or longer to grow a microorganism. In contrast the methods disclosed herein (for example when used on a PCR platform), are able to provide an indication of bacterial infection 2-3 hours from sample receipt, allowing prompt initiation of antibiotics. Since the methods of the disclosure allow the prompt initiation of antibiotics, the additional combination of optional confirmatory steps need not unduly delay treatment.

Methods of the invention, such as those employing a blood test, may allow patients with suspected bacterial meningitis to be identified swiftly, and guide medical staff to commence appropriate treatment promptly. Furthermore, such methods allow patients without meningitis to avoid unnecessary antibiotics and be discharged from hospital quicker.

Bacterial meningitis patients can present with a range of clinical symptoms and signs, such as headache, neck-stiffness, and dislike of bright lights (photophobia). However, some or all of these symptoms can be absent in individual subjects. As demonstrated in the Experimental Results, the methods of the invention are able to distinguish proven bacterial meningitis from 'look-a-like' conditions, such as meningism or viral meningitis that may give rise to symptoms that may otherwise be mistaken for those of bacterial meningitis.

Currently, bacterial meningitis is diagnosed by; (i) an elevated number of white cells in the CSF and (ii) detection of bacteria (by bacterial culture or a bacteria specific nucleic acid amplification test [NAAT]). Patients with meningism often have clinically indistinguishable symptoms and signs to bacterial meningitis, but lumbar punctures conducted on such subjects demonstrate that there are none or very few white cells in the CSF (<4 cells/$\mu$l). Patients with viral meningitis, again can have clinically indistinguishable symptoms and signs to bacterial meningitis, but these cases lumbar punctures detect virus in the CSF (via viral specific NAAT).

The methods disclosed herein allow the detection of bacterial infections, such as bacterial meningitis, even in cases where the sample is collected after the subject has received antibiotics and/or other anti-microbial treatment. This is in contrast to conventional methods for detecting bacteria, whether by culture or by NAAT, which often show poor sensitivity (fail to detect true cases of bacterial infection) when patient samples are collected after anti-microbial treatment has been initiated.

The combinations of host biomarker gene pairs used in the methods of the invention have been deliberately selected using patient blood samples collected both before and after (up to 6 weeks) initiation of anti-microbial treatment. Thus the invention offers to reduce the risk of missing true cases of bacterial meningitis among patients who have previously begun treatment with antibiotics.

By measuring human biomarkers in blood, this invention offers to revolutionise, accelerate and improve diagnosis of bacterial infections, guide prompt provision of appropriate antibiotic management and enhance patient outcomes. This is of particular benefit in the case of bacterial infections of the CNS, such as bacterial meningitis.

In order to assist the understanding of the present invention, certain terms used herein will now be further defined in the following paragraphs.

### *Bacterial infection*

A "bacterial infection", for the purposes of the present disclosure, may be taken as referring to any undesired presence and/or growth of bacteria in a subject. Such undesired presence of bacteria may have a negative effect on the host subject's health and well-being.

While the term "bacterial infections" should not be taken as encompassing the growth and/or presence of bacteria which are normally present in the subject, for example in the digestive tract of the subject, it may encompass the pathological overgrowth of such bacteria.

Bacterial infections may be caused by the growth and/or presence of gram positive and/or gram negative bacteria.

Bacterial infections may be caused by a wide range of bacteria. Merely by way of example, bacteria which are typically associated with meningo-encephalitis in human subjects may include: *Streptococcus pneumoniae, Neisseria Meningitides. Haemophilus influenzae, Streptococcus agalactiae, Listeria monocytogenes, Escherichia coli* and *Myco-*

*bacterium tuberculosis.*

Other bacteria involved in human infection of the central nervous system, particularly when subjects have indwelling catheters include: *Staphylococcus aureus, Pseudomonas aeruginosa* and *Ureaplasma urealyticum.*

Bacteria involved in human infection outside the central nervous system include: *Moraxella catarrhalis, Clostridium perfringens, Neisseria gonorrheae, Chlamydia trachomatis, Helicobacter pylori, Campylobacter jejuni, Salmonella enterica, Enterococcus faecalis, Clostridum difficile, Staphylococcus saprophyticus, Treponema pallidum, , Haemophilus ducreyi, Mycoplasma pneumoniae, Chlamydia pneumoniae,* and *Legionella pneumophila.*

The inventors believe that the methods or medical uses of the invention are applicable to bacterial infections (as set out in the first or second aspects of the invention) in which the bacterial pathogens are selected from the relevant groups set out above.

The methods or medical uses disclosed herein are applicable to bacterial infection in any system, organ or area of the subject, such as but not limited to, gastrointestinal system, respiratory system, urinary system, the ocular system, the auditory system and the skin. The results have also demonstrated that bacterial infections such as sepsis (blood stream infection) and pneumonia (respiratory bacterial infection) may be detected using the methods described herein.

The methods and medical uses disclosed herein are believed to have utility in helping to diagnose bacterial infections of prosthetic implants. These may include implants in the head or other body compartments. Merely by way of suitable example of a prosthetic head implant, the Experimental Results demonstrate that methods of the disclosure are able to correctly distinguish bacterial infection from mechanical failure in ventriculo-peritoneal (VP) shunts, using either blood or CSF samples. It is anticipated the methods disclosed herein would work equally well using body fluid from other implants, such as CSF from external ventricular drains in the head, or synovial fluid from the joint space around prosthetic knee replacements.

Without detracting from the above, the methods and uses of the invention are particularly suitable for use in connection with bacterial infections in the central nervous system, such as bacterial meningitis.

### Bacterial meningitis

The term "bacterial meningitis", as used herein, refers to inflammation of the meninges caused by any form of bacteria. Merely by way of example, bacterial meningitis may be caused by bacteria selected from the group consisting of: *Streptococcus pneumoniae, Neisseria Meningitides. Haemophilus influenzae, Streptococcus agalactiae, Listeria monocytogenes* and *Escherichia coli.*

As illustrated by the results presented in the Experimental Results section, the methods of the invention are able to distinguish between bacterial meningitis and other disorders of the meninges, including meningism and viral meningitis. References in the present specification to detection or diagnosis of bacterial meningitis should be construed as allowing such distinctions to be made, in keeping with the specificity and selectivity of the methods discussed further herein.

### Detecting bacterial infections

The methods disclosed herein are methods for detecting bacterial infections in a subject. In the context of the present disclosure, the term "detecting" is taken as meaning determining the presence of a bacterial infection in a subject. Suitably, and as discussed further below, the methods of the invention may be used to detect bacterial meningitis.

By way of example, the method of the disclosure may be used to detect a bacterial infection that is associated with symptoms in a subject. Alternatively the methods of the disclosure may also be used to detect a bacterial infection that is essentially asymptomatic. Such asymptomatic bacterial infections may be infections that are at a very early stage of the disease.

The inventors believe that the assessment, in the methods of the disclosure, of expression of genes associated with the subject's host response to bacterial infections to provide information regarding the presence of this condition confers notable advantages. Without wishing to be bound by any hypothesis, one such advantage may be that target molecules indicative of expression of such genes are found at detectable levels at earlier time points than are potential markers expressed by the bacteria associated with the infection. While levels of expression of bacterial genes may be difficult to detect, especially during the early stages of bacterial infections, the expression of host genes may be readily assessed. The greater abundance of such target molecules indicative of expression of host response genes not only allows such assessments to be made at an earlier time, but may also improve the accuracy of the assessment.

### Detecting bacterial meningitis

The methods of the invention are methods are particularly suitable for use in detecting bacterial meningitis in a subject.

Once bacterial meningitis has been detected by a method of the invention an additional step may optionally be practiced, to confirm the detection. The additional step may comprise performing a further diagnostic test for bacterial meningitis in respect of the subject. Such a further diagnostic test may be used to identify the bacterial pathogen present, or to provide information regarding the susceptibility of the bacterial pathogen to antimicrobial agents.

Examples of such diagnostic tests that may be employed will be well known to those skilled in the art. Suitably the further diagnostic test may comprise a lumbar puncture. Bacteria collected as part of the further diagnostic test may be investigated by culturing or by NAAT as described elsewhere in the specification.

It will be appreciated that, even when a lumbar puncture is used to confirm the detection or diagnosis provided by a method of the invention, the use of the methods of the invention significantly reduces the number of incidences of lumbar puncture that are needed (since lumbar puncture may be avoided in those cases where the methods of the invention indicate that bacterial meningitis is not present).

*A subject*

The methods and medical uses are practiced in respect of a subject. The subject may be one requiring diagnosis or treatment for a bacterial infection, such as bacterial meningitis. Suitably the subject may be a human subject. The subject may be a patient undergoing medical care, or an individual requesting medical care.

In the methods disclosed herein a suitable subject may be one believed to have a bacterial infection, such as bacterial meningitis. For example, a suitable subject may have symptoms consistent with a bacterial infection, such as bacterial meningitis. In such cases the methods of the disclosure may be of use in definitively detecting or diagnosing the presence of a bacterial infection, such as bacterial meningitis. Alternatively a suitable subject may lack some or all symptoms consistent with a bacterial infection, such as bacterial meningitis. As considered elsewhere in this specification, such a subject may be substantially asymptomatic.

Alternatively, a suitable subject in the context of the disclosure, may be one believed to be at risk of developing a bacterial infection, such as bacterial meningitis. Such a subject may have been in contact with an individual suffering from a bacterial infection and consequently may be believed to be at elevated risk of developing a bacterial infection as a result of this contact.

The ability of the methods of the disclosure to detect or diagnose a bacterial infection, such as bacterial meningitis, in a timely manner, and without the need for invasive procedures such as lumbar puncture, may be of benefit to any of the groups of subjects identified above.

It will be appreciated that a subject who may gain benefit from the methods of the disclosure is one in whom a bacterial infection is determined to be present by the assessment and comparisons conducted as part of the methods of the first, second or sixth aspects of the invention.

*A sample representative of gene expression in a subject*

The methods of the invention make use of samples that are representative of gene expression in the subject in respect of whom the method is being practiced. In particular, the methods disclosed herein use samples representative of expression of host response genes involved in a subject's response to bacterial infection. Methods of the disclosure may make use of any sample which contains target molecules that provide a representation of such gene expression in the subject in question.

By way of example, the sample is a body fluid sample. Alternatively, suitable samples may include tissue samples, such as biopsies. In either case, a suitable sample (whether of a body fluid or tissue) may comprise biological cells from the subject that are involved in the host response to bacterial infection.

A suitable body fluid sample may include: a blood sample (for example, a whole blood sample, a blood plasma sample, or a serum sample); or a cerebrospinal fluid (CSF) sample, or a synovial fluid sample.

Blood samples are particularly suitable for use in the methods of the invention. The use of a blood sample (whether whole blood, plasma, or serum) is advantageous, since it is readily accessible. Additionally obtaining the sample is not associated with much less risk and patient discomfort than is the case for samples such as CSF.

Samples may be processed for the enrichment of target molecules. Suitable techniques for such enrichment may be determined with reference to the nature of the sample, and of the target molecule in question. Generally, examples of suitable techniques (such as techniques for the isolation of biological cells from a sample, and the preparation of the cells to yield gene transcripts) will be well known to those skilled in the art.

*Target molecules*

Target molecules suitable for use in the method of the disclosure are any molecules which are representative of gene expression in the subject. Such target molecules may be representative of gene expression either directly or indirectly. By way of example, a suitable target molecule which is directly representative of gene expression may comprise an RNA transcript. A suitable target molecule which is indirectly representative of gene expression may comprise a protein encoded by the gene.

It will be appreciated that the selection of the type of target molecule to be used in a method of the invention should be considered in connection with the nature of the sample representative of gene expression. In the case of a sample that contains host cells, a suitable target molecule may be either directly indicative of gene expression (such as an RNA transcript), or indirectly indicative of gene expression (such as a protein encoded by an expressed gene). In the case of a sample that is essentially acellular, the use of target molecules that are indirectly indicative of gene expression may be preferred, since such target molecules (an in particular protein examples of such target molecules) may be shed into the sample, even in the absence of biological cells.

By the same token, the nature of the target molecule, and therefore the nature of the sample representative of gene expression, may be chosen in order to be consistent with use in a preferred assaying system.

## Assaying

When practicing the method of the disclosure, the sample is assayed to determine the relative abundance of the target molecules of interest. Generally, techniques suitable for assaying gene expression in order to provide information regarding relative abundance will be known to the person skilled in the art. As set out above, a suitable assay technique may be chosen with reference to the nature of the sample and the target molecules.

Merely by way of example, gene expression can be measured directly by techniques that allow the detection and quantification of RNA target molecules, such as RT-PCR, real-time PCR, Northern blot, RNA sequencing (RNA-seq) and RNA microarray.

Gene expression can be measured indirectly, by techniques that allow the detection and quantification of protein target molecules, such as ELISA, radioimmunoassay, immunoprecipitation, Western blot and mass spectrometry. Other suitable techniques for assaying proteins may be known to the person skilled in the art.

Also disclosed herein, an assay may allow the relative abundance of multiple sets of target molecules to be determined within a single reaction. An assay meeting such requirements may be referred to as a multiplex assay. Suitably a multiplex assay may allow the relative abundance of all requisite target molecules to be determined within a single reaction mixture (a "single tube" multiplex assay). Single tube multiplex assays may be particularly suitable for determining the relative abundance of mRNA transcript target molecules within a sample.

### Data indicative of relative abundance of target molecules

The methods disclosed herein involve obtaining data indicative of the relative abundance of relevant target molecules. For the purposes of the present invention, such "data indicative of relative abundance" may be taken as encompassing any data that are capable of being used in determining the presence of a bacterial infection, such as bacterial meningitis, in a subject. In a suitable disclosure the data provide a quantitative indication of relative abundance of expression of members of gene pairs, for example with reference to relevant target molecules.

### Genes and gene pairs suitable for use in the methods of the invention

The methods disclosed herein involve obtaining data indicative of the relative abundance of target molecules indicative of the expression of at least one of the gene pairs referred to in the disclosure. These gene pairs are made up of first and second host response genes.

The list of genes from which the gene pairs referred to herein are composed are set out in Table 1, which also provided details of the products encoded by these genes. Particular groups of gene pairs of interest that may be usefully employed in the methods of the are set out in Tables 2 to 5.

The expression of the genes and gene pairs is investigated by assaying the sample for target molecules indicative of the expression of such host response genes, as referred to above.

### *Table 2*

The methods disclosed herein involve obtaining data indicative of the relative abundance of target molecules indicative of the expression of at least at least one gene pair from the group consisting of: ELANE and IF144L; CTSG and IFI44L; ELANE and S1PR5; and CTSG and S1PR5. This group of gene pairs is set out in Table 2.

A method of the disclosure may involve obtaining data indicative of the relative abundance of target molecules indicative of the expression of at least two, or at least three of the gene pairs set out in Table 2, and determining the presence of a bacterial infection in the subject in dependence on the data in respect of each pair for which data is obtained. Suitably, a method of the disclosure may involve obtaining data indicative of the relative abundance of target molecules indicative of the expression of all four of the gene pairs set out in Table 2, and determining the presence of a bacterial infection in the subject in dependence on the data in respect of all four of these pairs.

### Table 3

For illustrative purposes, a method of the disclosure involves obtaining data indicative of the relative abundance of target molecules indicative of the expression of one, two, three, four, five, six, seven, eight, nine, ten, eleven, twelve or thirteen pairs of genes selected from the group consisting of: ELANE and IFI44L; CTSG and IFI44L; ELANE and S1PR5; CTSG and S1PR5; GRK6 and TXLNA; DUSP1 and IFI44L; APMAP and KMT2A; DUSP1 and HP1BP3; ,FLOT1 and NMT1, APMAP and HUWE1, ELANE and SIGLEC1; ELANE and IF127; and DUSP1 and NMT1. This group of gene pairs is set out in Table 3.

Suitably, a method disclosed herein may involve obtaining data indicative of the relative abundance of target molecules indicative of the expression of at least one, at least two, at least three, at least four, at least five, at least six, at least seven, at least eight, at least nine, at least ten, at least eleven, or at least twelve of the gene pairs set out in Table 3, and determining the presence of a bacterial infection in the subject in dependence on the data in respect of each pair for which data is obtained.

A method disclosed herein involves obtaining data indicative of the relative abundance of target molecules indicative of the expression of two, up to three, up to four, up to five, up to six, up to seven, up to eight, up to nine, up to ten, up to eleven, or up to twelve of the gene pairs set out in Table 3, and determining the presence of a bacterial infection in the subject in dependence on the data in respect of each pair for which data is obtained.

In a suitable example, a method of the disclosure may involve obtaining data indicative of the relative abundance of target molecules indicative of the expression of all thirteen of the gene pairs set out in Table 3, and determining the presence of a bacterial infection in the subject in dependence on the data in respect of all thirteen of these gene pairs.

### Table 4

In a further example, the method of the disclosure involves obtaining data indicative of the relative abundance of target molecules indicative of the expression of one, two, three, four, five or six pairs of genes selected from the group consisting of: GRK6 and TXLNA; DUSP1 and IFI44L; APMAP and KMT2A; DUSP1 and HP1BP3; APMAP and HUWE1; and DUSP1 and NMT1. This group of gene pairs is set out in Table 4.

Suitably, a method disclosed herein may involve obtaining data indicative of the relative abundance of target molecules indicative of the expression of at least one, at least two, at least three, at least four, or at least five of the gene pairs set out in Table 4, and determining the presence of a bacterial infection in the subject in dependence on the data in respect of each pair for which data is obtained.

In a suitable example a method of the disclosure involves obtaining data indicative of the relative abundance of target molecules indicative of the expression of two, up to three, up to four, or up to five of the gene pairs set out in Table 4, and determining the presence of a bacterial infection in the subject in dependence on the data in respect of each pair for which data is obtained.

In a suitable example a method of the disclosure may involve obtaining data indicative of the relative abundance of target molecules indicative of the expression of all six of the gene pairs set out in Table 4, and determining the presence of a bacterial infection in the subject in dependence on the data in respect of all six of these gene pairs.

### Table 5

In a suitable example, the method of the disclosure involves obtaining data indicative of the relative abundance of target molecules indicative of the expression of one, two, three, four, five, six, seven, eight, nine, or ten pairs of genes selected from the group consisting of: ELANE and IFI44L; CTSG and IFI44L; ELANE and S1PR5; CTSG and S1PR5; GRK6 and TXLNA; DUSP1 and IFI44L; APMAP and KMT2A; DUSP1 and HP1BP3, ELANE and SIGLEC1; and ELANE and IFI27. This group of gene pairs is set out in Table 5.

Suitably, a method of the disclosure may involve obtaining data indicative of the relative abundance of target molecules indicative of the expression of at least one, at least two, at least three, at least four, at least five, at least six, at least seven, at least eight, or at least nine of the gene pairs set out in Table 5, and determining the presence of a bacterial infection in the subject in dependence on the data in respect of each pair for which data is obtained.

In a suitable example a method of the disclosure involves obtaining data indicative of the relative abundance of target molecules indicative of the expression of two, up to three, up to four, up to five, up to six, up to seven, up to eight, or up to nine of the gene pairs set out in Table 5, and determining the presence of a bacterial infection in the subject in dependence on the data in respect of each pair for which data is obtained.

In a suitable example a method of the disclosure may involve obtaining data indicative of the relative abundance of target molecules indicative of the expression of all ten of the gene pairs set out in Table 5, and determining the presence of a bacterial infection in the subject in dependence on the data in respect of all ten of these gene pairs.

In a suitable example, a method of the disclosure may be based upon the use of data only in respect of genes, or gene pairs, set out in the preceding paragraphs. For example, a method of the invention may make use only of data in respect of the set of gene pairs set out in Table 2. Suitably, a method of the disclosure may make use only of data in respect of the set of gene pairs set out in Table 3. In a suitable example a method of the disclosure may make use only of data in respect of the set of gene pairs set out in Table 4. Alternatively a method of the disclosure may make use only of data in respect of the set of gene pairs set out in Table 5.

In another suitable example, the methods of the disclosure may employ one or more additional gene pairs beyond those set out in Table 3.

## Determining the presence of a bacterial infection

Suitably, in a method of the invention, the step of determining comprises using the data to calculate one or more functions, where the presence of a bacterial infection in the subject is determined to be present if one or more of the calculated functions satisfies a predetermined condition.

In a suitable embodiment, the one or more functions includes a logical function. In another suitable embodiment, the one or more functions includes a linear function. Examples of such embodiments are set out in the Experimental Results below.

Suitably the function is a linear discriminant function. An example of a suitable linear discriminant function that may be employed in the methods of the invention is described in the Experimental Results section.

Suitably the predetermined condition is satisfied if the calculated linear discriminant function exceeds a predetermined threshold. An example of a suitable predetermined threshold that may be employed with reference to a linear discriminant function is described in the Experimental Results section.

## Treatment of bacterial infections, such as bacterial meningitis

The present disclosure considers the treatment of bacterial infections, such as bacterial meningitis not only in the methods of treatment and medical uses described herein, but also in certain embodiments of the methods of detecting or diagnosing bacterial meningitis.

Treatment of bacterial infections, such as bacterial meningitis, in the context of the present disclosure will generally involve the provision of antibacterial agents to a subject requiring such treatment. Suitable antibacterial agents include antibiotics such as those selected from the group consisting of: Third generation cephalosporins (e.g. ceftriaxone, cefotaxime), ampicillin, penicillin G, meropenem, fluoroquinolone, trimethoprim-sulfamethoxazole, and chloramphenicol.

Suitably an antibacterial agent for use in accordance with this aspect of the invention may be selected with reference to the nature of the bacteria giving rise to bacterial meningitis, to be treated.

For illustrative purposes, in the case of a bacterial infection with *Streptococcus pneumoniae* an antibacterial agent for use in accordance with this aspect of the invention may be selected from the group of: vancomycin plus a third-generation cephalosporin; ceftriaxone or cefotaxime; Meropenem (C-III), fluoroquinolonec (B-II).

Thus, in the case of a bacterial infection with *Neisseria meningitidis* an antibacterial agent for use in accordance with this aspect of the disclosure may be selected from the group of: a third-generation cephalosporin; Ceftriaxone; Penicillin G, ampicillin, chloramphenicol, fluoroquinolone, aztreonam.

Thus, in the case of a bacterial infection with *Listeria monocytogenes* an antibacterial agent for use in accordance with this aspect of the disclosure may be selected from the group of: Ampicillin (optionally with addition of an aminoglycoside) or penicillin G (optionally with addition of an aminoglycoside) Trimethoprim-sulfamethoxazole, meropenem (B-III).

Thus, in the case of a bacterial infection with *Streptococcus agalactiae* an antibacterial agent for use in accordance with this aspect of the disclosure may be selected from the group of: Ampicillind or penicillin G (optionally with addition of an aminoglycoside) Third-generation cephalosporins (B-III).

Thus, in the case of a bacterial infection with *Haemophilus influenzae* an antibacterial agent for use in accordance with this aspect of the disclosure may be selected from the group of: Third-generation cephalosporin, Ceftriaxone; (A-I) Chloramphenicol, cefepime (A-I), meropenem (A-I), fluoroquinolone.

Thus, in the case of a bacterial infection with *Escherichia coli* an antibacterial agent for use in accordance with this aspect of the disclosure may be selected from the group of: Third-generation cephalosporin, ceftriaxone, (A-II) Cefepime, meropenem, aztreonam, fluoroquinolone, trimethoprim-sulfamethoxazole.

The invention will now be further described with reference to the accompanying Experimental Results and Figures, in which:

Figure 1 is a scatter plot of overall relative abundance ratio (formulated via discriminant rule) for 4 transcript host response test among proven bacterial meningitis compared to others (meningism or viral meningitis [n=10 per group]).

X-axis - patient groups. Whole-blood transcript abundance was measured in duplicate in each patient via qPCR.

Y-axis - overall relative abundance ratio. Nominal units. Median and Inter-quartile range exhibited.

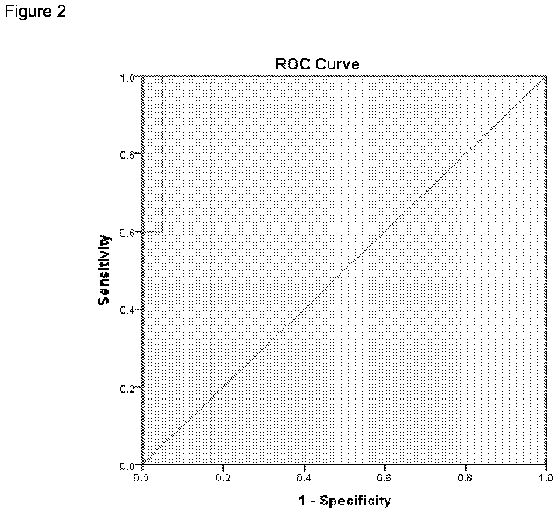

Figure 2 is a receiver operator curve (ROC) for the 4 transcript host response test, measured via qPCR, using patients with proven bacterial meningitis (n=10) compared to others (meningism or viral meningitis [n=20]). This is based on data from Figure 1.

Figure 3 is a scatter plot of overall relative abundance ratio (formulated via discriminant rule) for 4 transcript host response test among proven bacterial meningitis (n=13) compared to others (meningism or viral meningitis [n=88]).

X-axis - patient groups. Whole-blood transcript abundance was measured via qPCR in an independent sample set (n=101).

Y-axis - overall relative abundance ratio (formulated via discriminant rule). Nominal units. Median and Inter-quartile range exhibited.

Note: A low proportion of bacterial meningitis patients (13% [13/101]) was used in the sample set to reflect the prevalence of bacterial meningitis observed among adults with suspected meningitis in the UK.

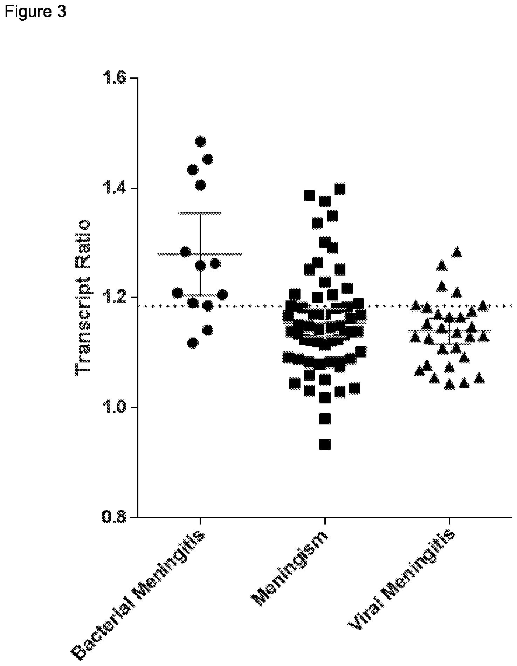

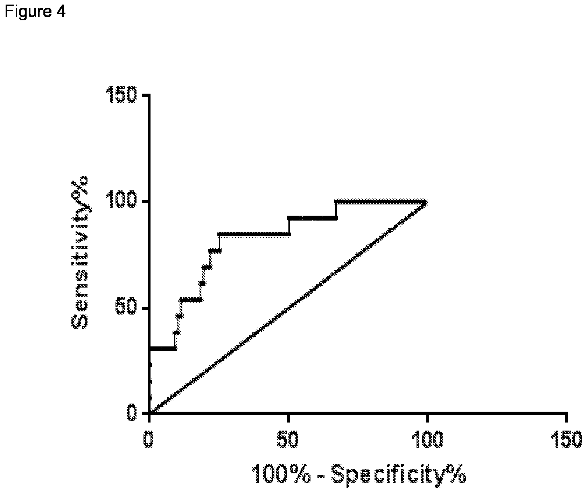

Figure 4 is a receiver operator curve (ROC) for the 4 transcript host response test, measured via qPCR, using patients with proven bacterial meningitis (n=13) compared to others (meningism or viral meningitis [n=88]). This is based on data from Figure 3.

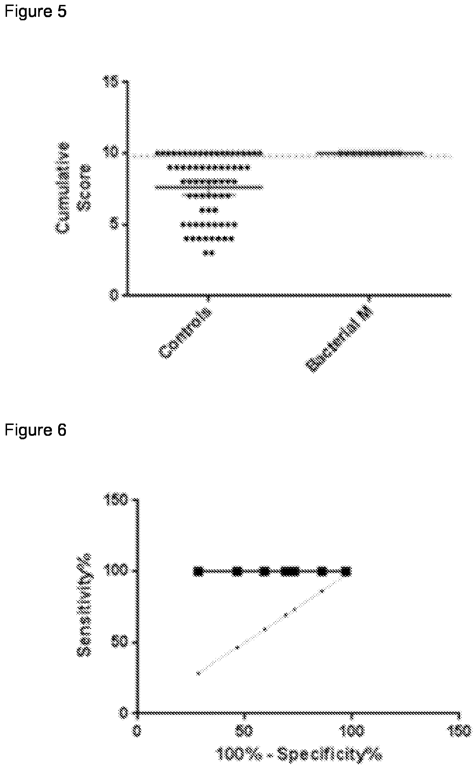

Figure 5 is a scatter-plot of cumulative score for 12 transcript host response test. Transcript abundance measured via qPCR, using patients with proven bacterial meningitis (n=10) compared to others (meningism or viral meningitis [n=71]). Relative ratio abundances from pairs of markers (n=10) were used to provide a cumulative score.

Figure 6 shows a receiver operator curve (ROC) for the 12 transcript host response test. Transcript abundance measured via qPCR, using patients with proven bacterial meningitis (n=10) compared to others (meningism or viral meningitis [n=71]). The ROC is based on data from Figure 5.

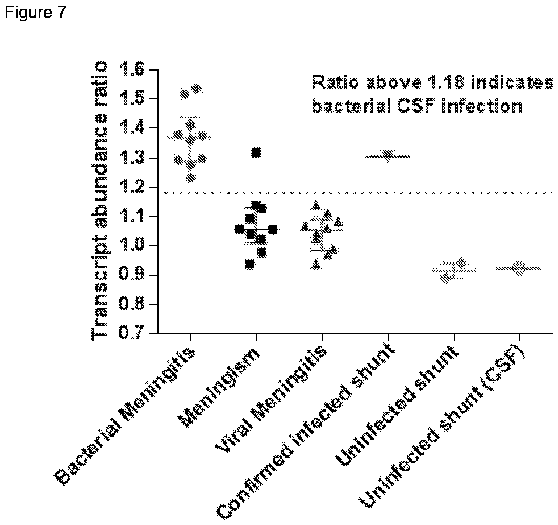

Figure 7 is a scatter plot of relative abundance ratio (formulated via discriminant rule) for 4 transcript host response test among proven bacterial meningitis and proven ventriculo-peritoneal (VP) shunt infection. The plot shows results for proven bacterial meningitis (n=10) compared to others (meningism or viral meningitis [n=20]). In addition the plot shows results for blood from patient with confirmed ventriculo-peritoneal (VP) shunt bacterial infection (n=1); blood from patient with confirmed mechanically blocked VP shunt (n=1) with additional sample from RNA extracted from CSF of the same patient.

X-axis - patient groups. Whole-blood transcript abundance was measured via qPCR in an independent sample set (n=101).

Y-axis - overall relative abundance ratio (formulated via discriminant rule). Nominal units. Median and Inter-quartile range exhibited.

## EXPERIMENTAL RESULTS

### 1 Methods

#### 1.1 Patient selection

The blood host response assay distinguishes confirmed bacterial CSF infection (positive culture or pathogen specific PCR in CSF; or CSF pleocytosis and positive culture or pathogen specific PCR in blood) from look-a-like conditions; namely meningism or viral meningitis (patients with signs and symptoms of infection but sterile CSF; or viral specific PCR in CSF).

Discriminatory markers for proven bacterial meningitis were initially identified through whole-genome transcriptomic analysis using blood samples from suspected adult meningitis patients (n=30) recruited through UK hospitals.

These markers were then re-assessed using transcript abundance meta-data compiled from previous microarray studies among child and adult patients with proven bacterial meningitis, meningism, viral meningitis, encephalitis from UK, Nepal, India, Malawi, Vietnam populations with and without HIV co-infection (n=180). Markers that continued to distinguish bacterial meningitis from others, were then tested using a quantitative PCR (qPCR) platform re-using patient samples (n=30). Markers that remained highly discriminatory for bacterial meningitis were then validated in an independent set of UK adult patients (n=101) with suspected meningitis via qPCR.

#### 1.2 Sample collection

Venous whole-blood samples (2.5mls) were collected into RNA stabilising tubes (Paxgene) during acute hospital admission (before or after antibiotic treatment had started) in consenting subjects.

### 1.3 RNA extraction

Total RNA was extracted using Blood RNA Extraction kits (Paxgene) following the manufacturers' instructions.

### 1.4 Gene-expression Microarrays

Total RNA (500ng per sample) was amplified and labelled using Low Input Quick Amp. Labelling kits (Agilent). Patient sample derived amplified RNA was labelled with Cyanine-3. Control human amplified RNA (Universal RNA) was labelled with Cyanine-5. Labelled RNA (200ng) was hybridised to SurePrint G3 GE 8x60K (Design ID 030495) human-specific microarrays following the manufacturers' instructions (Agilent Technologies). Arrays were scanned using Agilent G2505C scanner (Agilent Technologies). Raw fluorescent intensity was measured and initial quality control assessment undertaken using Agilent Feature Extraction software (FE 10.5.1.1). Transcript data were processed as previously described. Transcripts exhibiting a statistically significant difference in relative transcript abundance between proven bacterial meningitis cases and others were identified based on the fold change and false detection rate (FDR) output from SAM 4.0. (http://www-stat.stanford.edu/~tibs/SAM).

### 1.5 Quantitative PCR

Complimentary DNA (cDNA) was synthesised from total RNA (500ng per sample) using Retroscript II kit (Ambion). Quantitative PCR (qPCR) was performed with 50ng of amplified RNA DNA using TaqMan Gene Expression Assays (Applied Biosystems) in a BioRad real-time qPCR system following the manufacturer's assay and thermo-cycler set-up instructions. Individual single-plex assays (i.e. one assay per biomarker per well) were undertaken. Relative target transcript abundance between markers was quantified using the $\delta\delta$CT method.

### 1.6 Relative transcript abundance analysis

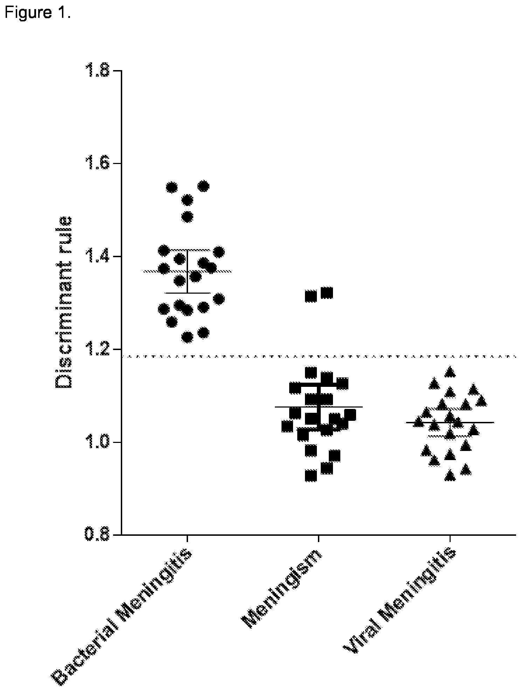

The relative transcript abundance for a set of 4 discriminatory genes (Table 2) in the microarray data was used to construct a linear discriminant function between proven bacterial (n=10) and others (n=20). The resulting discriminant rule predicted bacterial, if D>1.18, where D = 1.653*log (gene A) - 57.317* log(gene B) - 0.577*log (gene C) + 44.711*log (gene D) (Figure 1). These genes were then validated using qPCR in the later sets of patient samples (Figures 3 & 4).
By way of further example, identification of the sample being as indicative of bacterial meningitis by relative transcript marker abundances can also be arrived at using different equations (which can include logical operands). An example of such an equation is as follows:
1 [Bacterial Meningitis] = IF(AND(Gene A>Gene B*1.21, Gene C>Gene D*0.89,Gene B>Gene C*0.97),1,0)

$$D = (1.03*\log(1/Gene\ A+GeneB)/(1/GeneC+GeneD))$$

Using additional discriminatory markers identified during the meta-data analysis (Table 6), the marker set was optimised using combinations of 11 additional transcripts (Table 3). In these marker sets relative ratio abundance from pairs of markers (up to 13 pairs) were used to provide a cumulative score. Further analysis of the results achieved using genes set out in Table 3 showed markers could be dropped to produce two refined marker sets without any significant drop in marker set performance (Table 4 and Table 5). All of these marker sets exhibited improved sensitivity compared to the group set out in Table 2.

### 2 Results

The 4 transcript host response test measured via qPCR among 30 patient samples' (samples re-used from original microarray study) exhibited 100% (10/10) sensitivity, 95% (19/20) specificity and an accuracy of 97% (29/30).
The 4 transcript host response test (Table 2) measured via qPCR among 101 patient samples' (independent sample set from microarray studies) exhibited 85% (11/13) sensitivity, 74% (65/88) specificity and an accuracy of 75% (76/101).
The 15 transcript host response test (Table 3) measured via qPCR among 81 patient samples' (independent sample set from microarray studies) exhibited 100% (10/10) sensitivity, 72% (51/71) specificity and an accuracy of 75% (61/81)
The 9 transcript host response set (Table 4) measured via qPCR among 102 patient samples' (independent sample set from microarray studies) exhibited 91% (10/11) sensitivity, 74% (68/91) specificity and an accuracy of 76% (78/102).
The 12 transcript host response test (Table 5) measured via qPCR among 81 patient samples' (independent sample set from microarray studies) exhibited 100% (10/10) sensitivity, 72% (51/71) specificity and an accuracy of 75% (61/81) - Figure 5.

**TABLES OF GENES**

**Table 1**

| Gene | Encodes |
|---|---|
| ELANE | neutrophil elastase |
| IFI44L | Interferon-induced protein 44-like |
| CTSG | Cathepsin G |
| S1PR5 | sphingosine-1-phosphate receptor 5 |
| GRK6 | G protein-coupled receptor kinase 6 |
| TXLNA | taxilin alpha |
| DUSP1 | dual specificity phosphatase 1 |
| APMAP (formerly C20orf3) | adipocyte plasma membrane associated protein |
| KMT2A | lysine (K)-specific methyltransferase 2A |
| HP1BP3 | heterochromatin protein 1, binding protein 3 |
| SIGLEC1 | sialic acid binding Ig-like lectin 1, sialoadhesin |
| IFI27 | interferon, alpha-inducible protein 27 |
| NMT1 | N-myristoyltransferase 1 |
| FLOT1 | flotillin 1 |
| HUWE1 | HECT, UBA and WWE domain containing 1, E3 ubiquitin protein ligase |

**Table 2**

| First gene | Second gene |
|---|---|
| ELANE | IFI44L |
| CTSG | IFI44L |
| ELANE | S1PR5 |
| CTSG | S1PR5 |

**Table 3**

| First gene | Second gene |
|---|---|
| ELANE | IFI44L |
| CTSG | IFI44L |
| ELANE | S1PR5 |
| CTSG | S1PR5 |
| GRK6 | TXLNA |
| DUSP1 | IFI44L |
| APMAP | KMT2A |

(continued)

| First gene | Second gene |
|---|---|
| DUSP1 | HP1BP3 |
| FLOT1 | NMT1 |
| APMAP | HUWE1 |
| ELANE | SIGLEC1 |
| ELANE | IFI27 |
| DUSP1 | NMT1 |

**Table 4**

| First gene | Second gene |
|---|---|
| GRK6 | TXLNA |
| DUSP1 | IFI44L |
| APMAP | KMT2A |
| DUSP1 | HP1BP3 |
| APMAP | HUWE1 |
| DUSP1 | NMT1 |

**Table 5**

| First gene | Second gene |
|---|---|
| ELANE | IFI44L |
| CTSG | IFI44L |
| ELANE | S1PR5 |
| CTSG | S1PR5 |
| GRK6 | TXLNA |
| DUSP1 | IFI44L |
| APMAP | KMT2A |
| DUSP1 | HP1BP3 |
| ELANE | SIGLEC1 |
| ELANE | IFI27 |

**Table 6**

| GRK6 | TXLNA | DUSP1 | APMAP | KMT2A |
|---|---|---|---|---|
| HP1BP3 | FLOT1 | NMT1 | HUWE1 | SIGLEC1 |
| IFI27 | ITGA4 | METTL9 | DHRS7 | LRPAP1 |
| DEFA8P | LIPN | HP | HLA-DPA1 | JUNB |
| SF1 | IRS2 | TUBA4A | IFIT2 | ATM |
| MEF2A | IMPA2 | BIN1 | PLP2 | MADD |
| TRIM26 | CNPY3 | RAF1 | RHOG | PRKCD |

**EP 3 365 462 B1**

**Claims**

1. A method of detecting a bacterial infection of the blood stream or central nervous system in a subject, the method comprising:

   • assaying a blood sample representative of gene expression in the subject to obtain data indicative of the relative abundance ratio of target molecules indicative of the expression of the gene pair: ELANE and IFI44L, and optionally at least one further gene pair from the group consisting of: CTSG and IFI44L; ELANE and S1PR5; CTSG and S1PR5; GRK6 and TXLNA; DUSP1 and IFI44L; APMAP and KMT2A; DUSP1 and HP1BP3; FLOT1 and NMT1; APMAP and HUWE1; ELANE and SIGLEC1; ELANE and IF127; and DUSP1 and NMT1;
   • wherein the target molecules are selected from: an mRNA target molecule comprising an RNA transcript of the relevant gene, and a protein target molecule comprising a protein encoded by the relevant gene; and
   • determining the presence of a bacterial infection of the blood stream or central nervous system in the subject in dependence on the data.

2. A method according to claim 1 comprising obtaining data indicative of the relative abundance ratio of target molecules in respect of each of the gene pairs in the group consisting of ELANE and IFI44L; CTSG and IFI44L; ELANE and S1PR5; and CTSG and S1PR5; GRK6 and TXLNA; DUSP1 and IFI44L; APMAP and KMT2A ; DUSP1 and HP1BP3; APMAP and HUWE1; or DUSP1 and NMT1.

3. A method according to any preceding claim, in which a bacterial infection is detected in the subject, further comprising conducting a further confirmatory test for the bacterial infection in the subject.

4. A method according to any preceding claim, wherein the bacterial infection of the central nervous system is caused by bacteria selected from the group consisting of: *Streptococcus pneumoniae, Neisseria meningitidis, Haemophilus influenzae; Streptococcus agalactiae; Listeria monocytogenes; Escherichia coli; Mycobacterium tuberculosis; and Staphylococcus aureus.*

5. A method according to any preceding claim, wherein the target molecule is an mRNA target molecule, and wherein the assay is selected from the group consisting of: RT-PCR, real-time PCR, Northern blot, RNA sequencing (RNA-seq) and RNA microarray.

6. A method according to any preceding claim, wherein the target molecule is a protein target molecule, and wherein the assay is selected from the group consisting of: ELISA, radioimmunoassay, immunoprecipitation, Western blot and mass spectrometry.

7. A method according to any preceding claim, wherein the subject has symptoms consistent with a bacterial infection such as bacterial meningitis, or wherein the subject lacks some or all symptoms consistent with a bacterial infection such as bacterial meningitis.

8. A method according to any preceding claim, wherein subject is believed to be at risk of developing bacterial meningitis.

9. A method according to any preceding claim, wherein the step of determining comprises using the data to calculate one or more functions, where the presence of a bacterial infection in the subject is determined to be present if one or more of the calculated functions satisfies a predetermined condition.

10. A method according to claim 9, wherein the one or more functions includes a logical function or a linear function.

11. A method according to claim 10, wherein the linear function is a linear discriminant function, and optionally wherein the predetermined condition is satisfied if the calculated linear discriminant function exceeds a predetermined threshold.

12. An antibacterial agent for use in the treatment of a bacterial infection of the central nervous system in a subject identified as having a bacterial infection of the central nervous system by a method of any preceding claim.

13. An antibacterial agent for use according to claim 12, wherein the bacterial infection is bacterial meningitis.

**Patentansprüche**

1. Verfahren zum Nachweis einer bakteriellen Infektion des Blutkreislaufs oder des Zentralnervensystems bei einem Subjekt, wobei das Verfahren umfasst:

   • Untersuchen einer Blutprobe, die für die Genexpression bei dem Subjekt repräsentativ ist, um Daten zu erhalten, die das relative Häufigkeitsverhältnis von Zielmolekülen anzeigen, die die Expression des Genpaars anzeigen: ELANE und IFI44L, sowie optional mindestens ein weiteres Genpaar aus der Gruppe bestehend aus: CTSG und IFI44L; ELANE und S1PR5; CTSG und S1PR5; GRK6 und TXLNA; DUSP1 und IFI44L; APMAP und KMT2A; DUSP1 und HP1BP3; FLOT1 und NMT1; APMAP und HUWE1; ELANE und SIGLEC1; ELANE und IFI27; und DUSP1 und NMT1;
   • wobei die Zielmoleküle ausgewählt sind aus: einem mRNA-Zielmolekül, das ein RNA-Transkript des relevanten Gens umfasst, und einem Protein-Zielmolekül, das ein von dem relevanten Gen codiertes Protein umfasst; und
   • Bestimmen des Vorliegens einer bakteriellen Infektion des Blutkreislaufs oder des Zentralnervensystems bei dem Subjekt in Abhängigkeit der Daten.

2. Verfahren nach Anspruch 1, umfassend ein Erhalten von Daten, die das relative Häufigkeitsverhältnis von Zielmolekülen in Bezug auf jedes der Genpaare in der Gruppe anzeigen, die aus ELANE und IFI44; CTSG und IFI44L; ELANE und S1PR5; und CTSG und S1PR5; GRK6 und TXLNA; DUSP1 und IFI44L; APMAP und KMT2A ; DUSP1 und HP1BP3; APMAP und HUWE1; oder DUSP1 und NMT1 besteht.

3. Verfahren nach einem der vorhergehenden Ansprüche, bei dem eine bakterielle Infektion bei dem Subjekt nachgewiesen wird, ferner umfassend ein Durchführen eines weiteren Bestätigungstests für die bakterielle Infektion bei dem Subjekt.

4. Verfahren nach einem der vorhergehenden Ansprüche, wobei die bakterielle Infektion des Zentralnervensystems durch Bakterien verursacht wird, die ausgewählt sind aus der Gruppe bestehend aus: *Streptococcus pneumoniae, Neisseria meningitidis, Haemophilus influenzae, Streptococcus agalactiae; Listeria monocytogenes; Escherichia coli; Mycobacterium tuberculosis;* und *Staphylococcus aureus*.

5. Verfahren nach einem der vorhergehenden Ansprüche, wobei das Zielmolekül ein mRNA-Zielmolekül ist und wobei der Assay ausgewählt ist aus der Gruppe bestehend aus: RT-PCR, Echtzeit-PCR, Northern Blot, RNA-Sequenzierung (RNA-seq) und RNA-Microarray.

6. Verfahren nach einem der vorhergehenden Ansprüche, wobei das Zielmolekül ein Proteinzielmolekül ist und wobei der Assay ausgewählt ist aus der Gruppe bestehend aus: ELISA, Radioimmunoassay, Immunpräzipitation, Western Blot und Massenspektrometrie.

7. Verfahren nach einem der vorhergehenden Ansprüche, wobei das Subjekt Symptome hat, die mit einer bakteriellen Infektion, wie bakterieller Meningitis, übereinstimmen, oder wobei dem Subjekt einige oder alle Symptome fehlen, die mit einer bakteriellen Infektion, wie bakterieller Meningitis, übereinstimmen.

8. Verfahren nach einem der vorhergehenden Ansprüche, wobei angenommen wird, dass das Subjekt einem Risiko ausgesetzt ist, eine bakterielle Meningitis zu entwickeln.

9. Verfahren nach einem der vorhergehenden Ansprüche, wobei der Schritt des Bestimmens ein Verwenden der Daten umfasst, um eine oder mehrere Funktionen zu berechnen, wobei das Vorliegen einer bakteriellen Infektion bei dem Subjekt als vorhanden bestimmt wird, wenn eine oder mehrere der berechneten Funktionen eine vorgegebene Bedingung erfüllen.

10. Verfahren nach Anspruch 9, wobei die eine oder mehreren Funktionen eine logische Funktion oder eine lineare Funktion beinhalten.

11. Verfahren nach Anspruch 10, wobei die lineare Funktion eine lineare Diskriminanzfunktion ist, und wobei optional die vorgegebene Bedingung erfüllt ist, wenn die berechnete lineare Diskriminanzfunktion einen vorgegebenen Schwellenwert überschreitet.

12. Antibakterielles Mittel zur Verwendung bei der Behandlung einer bakteriellen Infektion des Zentralnervensystems bei einem Subjekt, bei dem eine bakterielle Infektion des Zentralnervensystems durch ein Verfahren nach einem der vorhergehenden Ansprüche festgestellt wurde.

13. Antibakterielles Mittel zur Verwendung nach Anspruch 12, wobei die bakterielle Infektion eine bakterielle Meningitis ist.

**Revendications**

1. Méthode de détection d'une infection bactérienne de la circulation sanguine ou du système nerveux central chez un sujet, la méthode comprenant :

   • l'analyse d'un échantillon de sang représentatif de l'expression génique chez le sujet pour obtenir des données indiquant le rapport d'abondance relative des molécules cibles indiquant l'expression de la paire de gènes : ELANE et IFI44L, et éventuellement d'au moins une autre paire de gènes du groupe constitué par : CTSG et IFI44L ; ELANE et S1PR5 ; CTSG et S1PR5 ; GRK6 et TXLNA ; DUSP1 et IFI44L ; APMAP et KMT2A ; DUSP1 et HP1BP3 ; FLOT1 et NMT1 ; APMAP et HUWE1 ; ELANE et SIGLEC1 ; ELANE et IFI27 ; et DUSP1 et NMT1 ;
   • lesdites molécules cibles étant choisies parmi : une molécule cible d'ARNm comprenant un transcrit d'ARN du gène pertinent, et une molécule cible de protéine comprenant une protéine codée par le gène pertinent ; et
   • la détermination de la présence d'une infection bactérienne de la circulation sanguine ou du système nerveux central chez le sujet en fonction des données.

2. Méthode selon la revendication 1, comprenant l'obtention de données indiquant le rapport d'abondance relative de molécules cibles par rapport à chacune des paires de gènes dans le groupe constitué par : ELANE et IFI44L ; CTSG et IFI44L ; ELANE et S1PR5 ; et CTSG et S1PR5 ; GRK6 et TXLNA; DUSP1 et IFI44L ; APMAP et KMT2A ; DUSP1 et HP1BP3 ; APMAP et HUWE1 ; ou DUSP1 et NMT1.

3. Méthode selon l'une quelconque des revendications précédentes, une infection bactérienne étant détectée chez le sujet, comprenant en outre la réalisation d'un autre test de confirmation de l'infection bactérienne chez le sujet.

4. Méthode selon l'une quelconque des revendications précédentes, ladite infection bactérienne du système nerveux central étant provoquée par des bactéries choisies dans le groupe constitué par : *Streptococcus pneumoniae, Neisseria meningitidis, Haemophilus influenzae ; Streptococcus agalactiae ; Listeria monocytogenes ; Escherichia coli ; Mycobacterium tuberculosis ; et Staphylococcus aureus.*

5. Méthode selon l'une quelconque des revendications précédentes, ladite molécule cible étant une molécule cible d'ARNm, et ladite analyse étant choisie dans le groupe constitué par : la RT-PCR, la PCR en temps réel, le transfert de Northern, le séquençage d'ARN (RNA-seq) et une micropuce à ARN

6. Méthode selon l'une quelconque des revendications précédentes, ladite molécule cible étant une molécule cible de protéine, et ladite analyse étant choisie dans le groupe constitué par : un test ELISA, un dosage radio-immunologique, une immunoprécipitation, le transfert de Western et la spectrométrie de masse.

7. Méthode selon l'une quelconque des revendications précédentes, ledit sujet présentant des symptômes compatibles avec une infection bactérienne telle qu'une méningite bactérienne, ou ledit sujet ne présentant pas certains ou tous les symptômes compatibles avec une infection bactérienne telle qu'une méningite bactérienne.

8. Méthode selon l'une quelconque des revendications précédentes, ledit sujet étant suspecté de présenter un risque de développer une méningite bactérienne.

9. Méthode selon l'une quelconque des revendications précédentes, ladite étape de détermination comprenant l'utilisation des données pour calculer une ou plusieurs fonctions, où la présence d'une infection bactérienne chez le sujet est déterminée comme étant présente si une ou plusieurs des fonctions calculées satisfont à une condition prédéfinie.

10. Méthode selon la revendication 9, lesdites une ou plusieurs fonctions comprenant une fonction logique ou une fonction linéaire.

**11.** Méthode selon la revendication 10, ladite fonction linéaire étant une fonction discriminante linéaire, et éventuellement ladite condition prédéfinie étant satisfaite si la fonction discriminante linéaire calculée dépasse un seuil prédéfini.

**12.** Agent antibactérien destiné à être utilisé dans le traitement d'une infection bactérienne du système nerveux central chez un sujet identifié comme étant atteint d'une infection bactérienne du système nerveux central par une méthode selon l'une quelconque des revendications précédentes.

**13.** Agent antibactérien destiné à être utilisé selon la revendication 12, ladite infection bactérienne étant une méningite bactérienne.

Figure 1.

Figure 2

ROC Curve

Figure 3

Figure 4

Figure 5

Figure 6

Figure 7

**REFERENCES CITED IN THE DESCRIPTION**

**Patent documents cited in the description**

- WO 2015155517 A
- US 20140323391 A